# EUROPEAN PATENT SPECIFICATION

(11) **EP 1 063 992 B1**
(45) Date of publication and mention of the grant of the patent: **11.05.2005**
(21) Application number: 99912614.7
(22) Date of filing: 18.03.1999
(51) Int. Cl.: A61K 31/415, A01N 43/48

(54) **SULFURPENTA FLUOROPHENYL PYRAZOLES FOR CONTROLLING ECTOPARASITIC INFESTATIONS**
SCHWEFELPENTAFLUOROPHENYLPYRAZOLE ZUR KONTROLLE VON EKTOPARASITENBEFALL
SULFURPENTA FLUOROPHENYL PYRAZOLES UTILES DANS LA LUTTE CONTRE LES INFESTATIONS PAR LES ECTOPARASITES

(30) Priority: 19.03.1998 US 78639 P; 21.07.1998 GB 9815802
(43) Date of publication of application: 03.01.2001
(73) Proprietor: MERCK & CO., INC., Rahway, New Jersey 07065 (US)
(72) Inventor: CHERN, Rey, T., Rahway, NJ 07065 (US); ZINGERMAN, Joel, R., Rahway, NJ 07065 (US); CLARK, Jeffrey, N., Rahway, NJ 07065 (US); DRAG, Marlene, D., Rahway, NJ 07065 (US)
(74) Representative: Horgan, James Michael Frederic
(86) International application number: PCT/US1999/005815
(87) International publication number: WO 1999/047139

(56) References cited:
- WO-A-97/12521
- WO-A-98/04530
- DE-A- 19 518 054
- US-A- 5 451 598
- US-A- 5 637 607
- 1984, CROOM HELM , LONDON & SYDNEY * page 65 - page 80 *
- Spinosad Technical Guide (Dow AgroSciences Inc.)
- 1989, SPRINGER VERLAG , NEW YORK * page 288 - page 310 *

## Description

### SUMMARY OF THE INVENTION

The present invention concerns a method for controlling ectoparasitic infestation using highly potent compounds, a long acting injectable formulation of such compounds which provides an unexpected long duration of efficacy, and a method for administering said compounds while avoiding or reducing the side effects that may occur with such compounds. Additional objects will become apparent from a reading of the following description.

### BACKGROUND OF THE INVENTION

Blood sucking parasitic infestation on animals, especially the infestation of pets by fleas, has been a continued problem in the art. Infestation of dogs and cats with fleas may cause local irritation or annoying scratching. Intense scratching can lead to open wounds that can become infected with bacteria.

Many different types of flea deterring or larvicide or adulticide treatments have been developed in attempts to rid animals of fleas. A variety of products have been marketed for controlling flea infestation in household pets such as dogs, cats, hamsters, etc. Most products contain harsh chemicals that can have serious consequences when used too often or in excess of recommended quantities. Typical of these chemicals are propoxur (o-isopropoxphenyl), methyl carbamate, diazinon, chlorpyrifos, d-limonene, cyano(3-phenoxypheny)methyl 4-chloro-alpha (1-methylethyl)benzeneacetate, pyrethrins, piperonyl butoxide and N-octyl bicycloheptane dicarboximide. Although these chemicals are generally effective against fleas if used carefully, they can often have serious side effects. Flea resistance to these compounds has also been seen in the field. Many products may excessively dry the skin or cause eczema or allergic reactions in some animals. Skin wounds caused by the animal's scratching at fleas can become infected and the infections are often aggravated by these chemicals. Many of these chemicals cannot be applied to the fur of animals, such as cats, that self-groom by licking the skin and fur. Persons applying these flea killing chemicals to animals must often be very careful to avoid excessive contact with them. Those grooming animals are advised to wear rubber gloves to avoid continuous contact with the chemicals. Care must be exercised to keep the chemicals out of the eyes and away from the mucus membranes of both the animal and the person applying them. Breathing vapor from the chemicals over long periods must also be avoided. Many of these chemicals are not rapidly biodegradeable and constitute an environmental hazard if misused. Thus, there is a continuing need for improved and fully effective materials that can be applied to fur bearing animals to eliminate fleas while being environmentally benign, and avoiding deleterious side effects.

1-(4-SF₅-phenyl)pyrazoles of formula I are known compounds reportedly to be useful as insecticides (see US Patent 5,451,598). They have not been disclosed as useful in controlling ectoparasites, such as flea and tick infestations in animals.

PCT Published Application WO97/12521 discloses ready-to-use solutions for topical application against fleas and ticks containing as active ingredient a compound of the formula 1: 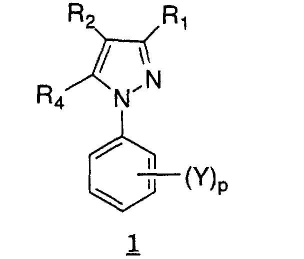 wherein Y may be halogen or SF₅, among others, and p may be from 1 to 5. The only active compound specifically exemplified in the claimed ready-to-use solution is the compound fipronil 2:

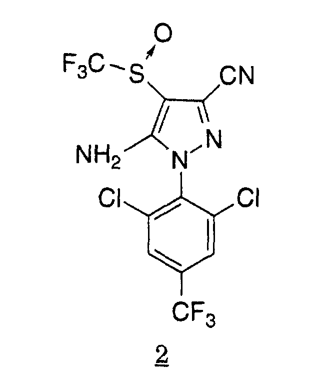

Fipronil is commercially available in topical formulations against fleas and ticks. It has been known to cause vomition in animals when administered systemically. Topical application of fipronil provides efficacy for not more than three months.

The present inventors have found that certain SF₅-phenylpyrazoles are highly potent agents against fleas and ticks. More particularly these SF₅-phenylpyrazoles exhibit higher potency than fipronil against fleas. The SF₅-phenylpyrazoles may be formulated for systemic administration to provide efficacy against fleas and/or ticks for extended period while avoiding or reducing the potential of vomition and/or other adverse reactions in treated animals.

### BRIEF DESCRIPTION OF THE DRAWINGS

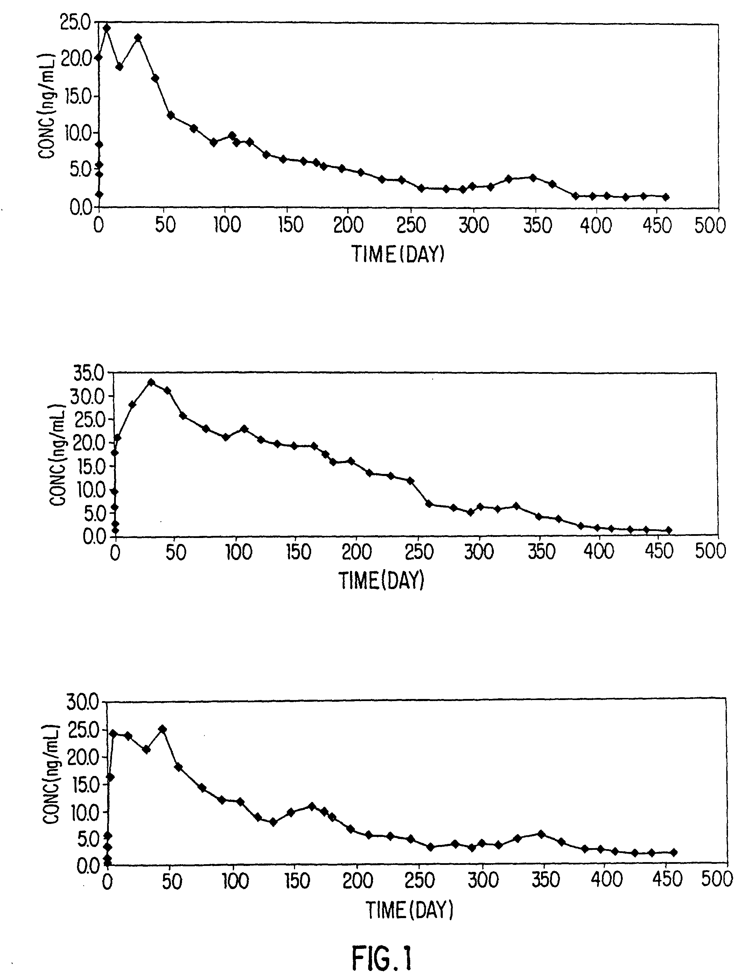

Figure 1 depicts plasma levels of 6-amino-3-cyano-1-(2,6-dichloro-4-sulfurpentafluorophenyl)-4-(trifluoromethylthio)pyrazole in dogs treated with the formulation of Example 1.

### DETAILED DESCRIPTION OF THE INVENTION

The present invention provides for a method of controlling ectoparasitic infestation in a companion animal which comprises administering to said animal a therapeutically effective amount of a compound of formula I: 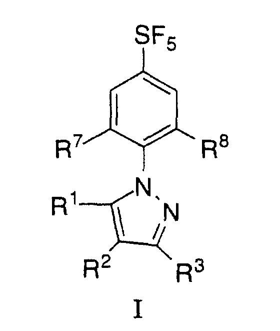 wherein
- R¹ is: hydrogen, halogen, or a group NR⁴R⁵;
- R² is: -S(O)ₙR⁶;
- R³ is: -CN or CX-NY¹Y²;
- R⁴ and R⁵ are: independently hydrogen or alkyl;
- R⁶ is: haloalkyl;
- R⁷ and R⁸ are: independently halogen;
- X is: O or S;
- Y¹ and Y²: are independently hydrogen, nitro, amino, or alkyl optionally substituted by halogen, cycloalkyl, formyl, C₂₋₇ alkanoyl, C₄₋₇ cycloalkylcarbonyl, C₂₋₇ alkoxycarbonyl, C₂₋₇ haloalkoxycarbonyl, aryl, or aromatic heterocyclic group; or
- Y¹ and Y²: together with the nitrogen to which they are attached form an aliphatic heterocyclic group containing from 4 to 8 ring atoms and optionally substituted by halogen or alkyl; or
- Y¹ and Y²: together form the group =CHY³; or
- Y¹ is: hydrogen and Y² is alkoxycarbonyl, alkylcarbonyl, optionally substituted aralkyl, or -S(O)nR⁶;
- Y³ is: alkyl, C₂₋₆ alkenyl, aryl, an aromatic heterocycle, or amino optionally substituted by halogen;
- n is: 0, 1 or 2; or
a pharmaceutically acceptable salt thereof.

Another aspect of the present invention provides a method of controlling ectoparasitic infestation in a companion animal while avoiding or reducing the potential of vomition and/or other adverse reactions which comprises administering systemically to said animal a therapeutically effective amount of a compound of formula I in a formulation that provides peak plasma level of the administered compound lower than about 100 ng/ml.

Another aspect of the present invention provides a pharmaceutical composition for injection which comprises:
(1) a compound of formula I;
(2) a biologically acceptable polymer; and
(3) a lipophilic solvent.

Another aspect of the present invention provides a method of controlling ectoparasitic infestation in a companion animal while avoiding or reducing the potential of vomition and/or other adverse reactions which comprises injecting said animal with a pharmaceutical composition comprising:
(1) a compound of formula I;
(2) a biologically acceptable polymer; and
(3) a lipophilic solvent.

In one subset of compounds of formula I R¹ is halogen (such as bromine) or a group NR⁴R⁵ (such as NH₂).

In another subset of compounds of formula I R³ is CN.

In another subset of compounds of formula I R² is S(O)ₙR⁶ wherein R⁶ is halomethyl (such as trifluoromethyl) or haloethyl (such as 1,1-difluoroethyl).

In another subset of compounds of formula I R⁷ and R⁸ are independently chloro.

In a preferred embodiment the ectoparasite to be controlled is flea or tick, more preferably flea.

In another preferred embodiment the compound of formula I is administered parenterally.

In another preferred embodiment the methods and pharmaceutical compositions of the present invention employ compounds of formula Ia: 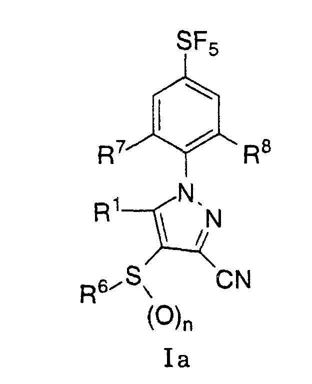 wherein
- R¹ is: halogen or NR4R5;
- R⁴ and R⁵ are: independently hydrogen or C₁₋₄ alkyl;
- R⁶ is: C₁₋₄ haloalkyl;
- R⁷ and R⁸ are: independently chloro or bromo; and
- n is: 0, 1 or 2.

A more preferred embodiment of the present methods and pharmaceutical compositions employs compounds of formula Ia wherein
- R¹ is: bromo or NR⁴R⁵;
- R⁴ and R⁵ are: independently hydrogen or methyl; and
- R⁶ is: halomethyl or haloethyl.

Representative compounds of formula I suitable for use in the present methods or pharmaceutical compositions include:
(1) 6-amino-3-cyano-1-(2,6-dichloro-4-sulfurpentafluorophenyl)-4-(trifluoromethylthio)pyrazole;
(2) 6-amino-3-cyano-1-(2,6-dichloro-4-sulfurpentafluorophenyl)-4-(trifluoromethylsulfinyl)pyrazole;
(3) 6-amino-3-cyano-1-(2,6-dichloro-4-sulfurpentafluorophenyl)-4-(trifluoromethylsulfonyl)pyrazole;
(4) 6-amino-3-cyano-1-(2,6-dichloro-4-sulfurpentafluorophenyl)-4-(1,1-difluoroethylthio)pyrazole; and
(5) 6-bromo-3-cyano-1-(2,6-dichloro-4-sulfurpentafluorophenyl)-4-(trifluoromethylthio)pyrazole.

In another preferred embodiment the pharmaceutical composition for injection further comprises a hydrophilic solvent.

The term "alkyl" as used herein includes straight or branched chain alkyl groups, preferably containing up to 6 carbon atoms. This applies also to alkyl moieties contained in other groups such as "haloalkyl" groups. The term "cycloalkyl" used herein refers to a carbocyclic ring suitably having from 3 to 10 and preferably from 3 to 7 carbon atoms in the ring.

The term "halogen" includes fluorine, chlorine, bromine and iodine.

The term "systemic administration" includes oral and parenteral administration.

The term "companion animal" includes dogs and cats.

The term "controlling" includes preventing (e.g. prophylactic use), treating, eradicating, ameliorating or otherwise reducing the severity of the condition being controlled.

The term "composition", as in pharmaceutical composition, is intended to encompass a product comprising the active ingredient(s), and the inert ingredient(s) that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of formula I and the other specified components that comprise the carrier.

The term "pharmaceutical composition" is intended to mean a composition suitable for use in veterinary medicine. The term "pharmaceutically acceptable" is intended to mean acceptable for use in animals, particularly in companion animals such as dogs and cats.

"Biologically acceptable polymer" can be any biologically acceptable polymer, such as a biologically acceptable polymer recognized in the art. For instance, the biologically acceptable polymer can have one or more or all of the following characteristics: be bioerodible by cellular action, biodegradable by action of non-living body fluid components, soften when exposed to heat but return to the original state when cooled and are capable of substantially dissolving or dispersing in a water-miscible carrier or solvent to form a solution or dispersion. Upon contact with an aqueous fluid and the polymer are capable of assisting in the formation of the film coated or encapsulated liquid. The kinds of polymers suitable for the present composition generally include any having the foregoing characteristics. Examples are polylactides, polyglycolides, polycaprolactones, polyanhydrides, polyamides, polyurethanes, polyesteramides, polyorthoesters, polydioxanones, polyacetals, polyketals, polycarbonates, polyorthocarbonates, polyphosphazenes, polyhydroxybutyrates, polyhydroxyvalerates, polyalkylene oxalates, polyalkylene succinates, poly(malicacid), poly(amino acids), poly(methyl vinyl ether), poly(maleic anhydride), chitin, chitosan, and copolymers, terpolymers, or combinations or mixtures therein. Polylactides, polycaprolactones, polyglycolides and copolymers thereof are preferred polymers, with poly(lactide-co-glycolide) copolymer ("PLGA") highly preferred.

"Poly(lactide-co-glycolide)" means a copolymer of lactic and glycolic acids having a lactide:glycolide ratio of from 95:05 to 50:50, preferably 75:25 to 65:35. The lactic acid can be d- or 1- or dl-. The copolymer may be a single copolymer of a mixture of copolymers within the above-defined parameters.

"Hydrophilic solvent" means water miscible solvents, preferably those when mixed with water in a ratio from 1:9 to 9:1 form a single-phase solution. For example, propylene glycol, polyglycols such as polyethylene glycol 200, polyethylene glycol 300 and polyethylene glycol 400, di(ethylene glycol)ethyl ether (Transcutol), isopropylidene glycerol (Solketal), dimethyl isosorbide, propylene carbonate, glycerol, glycerol formal, glycofural, pyrrolidones such as N-methyl pyrrolidone and 2-pyrrolidone, di(propyleneglycol) methyl ether, and mixtures thereof. Other solvents may also be useful as the hydrophilic solvent. For instance, the hydrophilic solvent can be a C₂ to C₆ alkanol (e.g., ethanol, propanol, butanol), acetone, alkyl esters such as methyl acetate, ethyl acetate, ethyl lactate, alkyl ketones such as methyl ethyl ketone, dialkylamides such as dimethylformamide, dimethyl sulfoxide, dimethyl sulfone, tetrahydrofuran, cyclic alkyl amides such as caprolactam, decylmethylsulfoxide, oleic acid, propylene carbonate, aromatic amides such as N,N-diethyl-m-toluamide, and 1-dodecylazacycloheptan-2-one. The hydrophilic solvent can be a mixture of solvents.

"Lipophilic solvent" means water immiscible solvents, preferably with a solubility in water of less than 10% at room temperature. Lipophilic solvent may be triethyl citrate, Miglyol 812, Miglyol 840, Crodamol GTCC, triacetin, triethyl citrate or benzyl benzoate; and additional lipophilic solvents may be used, e.g., hydrophobic rate modifying agents or plasticizers such as fatty acids, triglycerides, triesters of glycerol, oils such as castor oil, soybean oil or other vegetable oils or derivatives thereof such as epoxidized or hydrogenated vegetable oils such as epoxidized soybean oil or hydrogenated castor oil, sterols, higher alkanols (e.g., C₆ or higher), glycerin and the like. The lipophilic solvent can be a mixture of solvents.

Compounds of formula I are highly potent agents against ectoparasites, particularly fleas and ticks, and especially fleas. Thus compounds of formula I have utility for controlling ectoparasitic infestations in animals, particularly in companion animals such as dogs and cats. Preferably compounds of formula I are used for controlling flea infestation in dogs.

Compounds of formula I may be administered in formulations wherein the active compound is intimately admixed with one or more inert ingredients and optionally including one or more additional active ingredients. The compounds may be used in any composition known to those skilled in the art for systemic administration to animals. For application to animals to control ectoparasites, oral formulations, in solid or liquid or parenteral liquid, implant or depot injection forms may be used.

The optimum amount to be employed for best results will, of course, depend upon the particular compound employed, the species of animal to be treated, the route and formulation of administration, and the type and severity of parasitic infection or infestation. Generally good results are obtained with compounds of formula I when administered from about 0.1 to 50 mg per kg of animal body weight, such total dose being given at one time or in divided doses over a relatively short period of time such as 1-5 days.

Compounds of formula I may be administered orally in a unit dosage form such as a capsule, bolus or tablet including chewable tablet. These dosage forms are prepared by intimately and uniformly mixing the active ingredient with suitable finely divided diluents, fillers, disintegrating agents, and/or binders such as starch, lactose, talc, magnesium stearate, vegetable gums and the like. Such unit dosage formulations may be varied widely with respect to their total weight and content of the antiparasitic agent depending upon factors such as the type of host animal to be treated, the severity and type of infection and the weight of the host.

When the active compound is to be administered via an animal feedstuff, it is intimately dispersed in the feed or used as a top dressing or in the form of pellets or liquid which may then be added to the finished feed or optionally fed separately. Alternatively, feed based individual dosage forms may be used such as a chewable treat.

Compounds of formula I may be administered to animals parenterally, for example, by intraruminal, intramuscular, intravascular, intratracheal, or subcutaneous injection in which the active ingredient is dissolved or dispersed in a liquid carrier vehicle. Parenteral vehicle may be of the vegetable oil variety (e.g. peanut oil, cotton seed oil, propylene glycerol octanoate decanoate and the like). Other parenteral vehicles such as organic preparation using triacetin, benzyl benzoate, solketal, glycerol formal, propylene glycol, and aqueous parenteral formulations are also used. The active compound or compounds are dissolved or suspended in the parenteral formulation for administration; such formulations generally contain from 0.0005 to 20% by weight of the active compound. A preferred parenteral formulation is described hereinbelow.

Compounds of the present invention may be co-administered or used with other anthelmintic agents. These anthelmintic agents are meant to include, but not be restricted to, compounds selected from the avermectin and milbemycin class of compounds such as ivermectin, avermectin, abamectin, emamectin, eprinomectin, doramectin, milbemycin derivatives described in EPO 357460, EPO 444964 and EPO 594291, moxidectin, Interceptor™ and nemadectin. Additional anthelmintic agents include the benzimidazoles such as thiabendazole, cambendazole, parbendazole, oxibendazole, mebendazole, flubendazole, fenbendazole, oxfendazole, albendazole, cyclobendazole, febantel, thiophanate and the like. Additional anthelmintic agents include imidazothiazoles and tetrahydropyrimidines such as tetramisole-levamisole, butamisole, pyrantel, pamoate, oxantel or morantel.

Compounds of this invention may be co-administered or used in combination with fipronil (FRONTLINE™); or with an insect growth regulator with molt inhibiting activity such as lufenuron (PROGRAM™) and the like; or with ecdysone agonists such as tebufenozide and the like, which induces premature molt and causes feeding to cease; or with imidacloprid (ADVANTAGE™).

Compounds of this invention may be co-administered or used in combination with avermectin or milbemycin or doramectin derivatives such as those described in US Patent 5,015,630, WO 94/15944, WO95/22552.

Compounds of this invention may be co-administered or used in combination with cyclic depsipeptides that exhibit anthelmintic efficacy such as those described in WO96/11945, WO93/19053, WO93/25543, EP 626375, EP 382173, WO 94/19334, EP 382173 and EP 503538.

Compounds of this invention may be co-administered or used in combination with nodulisporic acid derivatives such as those described in PCT Application WO96/29073.

Compounds of this invention may be used in combination or be co-administered with derivatives and analogs of the general class of dioxomorpholine antiparasitic and anthelmintic agents as illustrated by WO 9615121; or with pyrethroids or organophosphates or insecticidal carbamates, such as those described in "Chemotherapy of Parasitic Diseases", Campbell, W.C. and Rew, R. S, Eds., 1986; or with derivatives and analogs of the general class of paraherquamide and macfortine anthelmintic agents, as well as oxazoline anthelmintics and insecticides such as those disclosed in US 5,478,855, US 4,639,771 and DE 19520936.

The co-administered compounds are given via routes and in doses that are customarily used for those compounds. These compounds may also be included as a second active ingredient in compositions containing the sulfurpentafluorophenylpyrazoles of the present invention.

The present inventors have further discovered that compounds of formula I may be administered systemically while avoiding or reducing the potential of vomition and/or other adverse reactions in treated animals when the peak plasma level of the administered active compound is kept below about 100 ng/ml. Depending on the specific compound, route of administration, formulation, the peak plasma level may be kept below the target level when the dose administered ranges from about 0.05 mg/kg to about 50 mg/kg. In general oral dosage of about 0.5 mg/kg to about 3 mg/kg and parenteral dosage of about 1 mg/kg to about 20 mg/kg may be used.

Another aspect of the present invention provides for a long-acting injectable composition containing a compound of formula I which provides efficacy in controlling ectoparasitic infestation in animals for at least six months after a single injection while avoiding or reducing the potential of vomition and/or other adverse reactions in treated animals. This long-acting injectable composition comprises: 1) a compound of formula I, 2) a biologically acceptable polymer, and 3) a lipophilic solvent. In a preferred embodiment, the biologically acceptable polymer is poly(lactide/glycolide) copolymer. In another preferred embodiment the composition further comprises a hydrophilic solvent.

In the long-acting injectable composition, poly(lactide/glycolide) copolymers with lactide/glycolide ratio in the range of 95/5 to 50/50, molecular weight in the range of 2,000 to 100,000 are suitable; preferably the lactide:glycolide ratio is from 75:25 to 65:35.

The lipophilic solvent includes but is not limited to triacetin, propylene glycerol octanoate decanoate, benzyl benzoate, ethyl oleate, other vegetable oils and derivatives, and triethyl citrate, and mixtures thereof. The hydrophilic solvent includes, but is not limited to, glycerol formal, glycofural, N-methyl pyrrolidone, 2-pyrrolidone, isopropylidene glycerol, di(propylene glycol) methyl ether, and mixtures thereof.

The active compound is present in the composition in amounts ranging from about 1% w/v to about 30% w/v; preferably 1 to 10%, and more preferably 5 to 10 %. The polymer is present in amount ranging from about 1 to about 20% w/v; preferably about 1 to about 10% w/v, and more preferably 5 to 10%. Preferably, the weight ratio of the copolymer to the active compound is less than or equal to 1:1; for example about 0.3:1 to 1:1. The lipophilic vehicle is present in about 10 to about 100% v/v, preferably from about 20 to about 90%, with the balance being made up of the hydrophilic vehicle.

The long-acting injectable composition may be prepared by dissolving all the solid ingredients in the vehicle under normal manufacturing conditions used for sterile injectable products. The present composition may contain additional inert substances commonly used in parenteral formulations including, but not limited to, antimicrobial agents, antioxidants, etc, as well as additional active ingredients such as those described above as being suitable for combination with compounds of formula I.

The long-acting injectable composition is capable of maintaining systemic drug concentration at a relatively constant level, and avoiding "burst" delivery, over a prolonged period of time that can be more than six months. Thus it is especially suitable for the delivery of compounds of formula I where sharp peak drug plasma levels, which may be over 100 ng/ml, are preferably avoided to minimize potential for vomition and/or other adverse reactions. Accordingly, the present invention further contemplates a method of controlling ectoparasitic infestation in a companion animal while avoiding or reducing the potential of vomition in the treated animals which comprises injecting said animal with a long-acting injectable composition as described above. The method is particularly useful for controlling flea infestation in dogs.

The following examples are provided to illustrate the invention.

### EXAMPLE 1

Preparation of long-acting injectable formulation containing 6-amino-3-cyano-1-(2,6-dichloro-4-sulfurpentafluorophenyl)-4-(trifluoromethylthio) pyrazole

Poly(DL-lactide/glycolide) 75/25 (PLGA, 0.25 g) was dissolved in sufficient glycerol formal to provide a 2.5 ml solution. In a separate flask poly(DL-lactide/glycolide) 75/25 (0.25 g) was dissolved in sufficient triacetin to provide a 2.5 ml solution. The two PLGA solutions were mixed well and added to a flask containing the active ingredient (0.50 g). The contents of the flask were mixed until the active ingredient dissolved, and the resulting solution was sterile filtered into a vial and sealed.

### EXAMPLE 2

Activity of 6-amino-3-cyano-1-(2,6-dichloro-4-sulfurpentafluorophenyl)-4-(trifluoromethylthio)pyrazole against fleas in dogs

Beagle dogs of both sexes 21 to 31 months old and weighing from 10 to 16 kg were used. Groups of three dogs each were treated with the same active compound in different formulations as follows:
Group 1: topical formulation containing 10% w/v of active compound, 5% w/v polyvinylpyrrolidone 30 and ethanol qs to 100%v/v dosed at 5 mg/kg.
Group 2: long acting parenteral formulation containing 10% w/v of the active compound, 25% w/v of sucrose octaacetate, 1% w/v of propylene glycol octanoate decanoate and triacetin qs 100% v/v dosed subcutaneously at 10 mg/kg.
Group 3: long acting parenteral formulation containing 10% w/v active compound, 10% w/v poly(DL-lactide/glycolide)75/25, and qs 100% v/v 1:1 glycerol formal/triacetin dosed at 10 mg/kg subcutaneously.
Control group of three untreated dogs was included.

Treatments were administered once on Day 0. On Day 1 animals all animals were infested with approximately 100 fleas. Animals were combed and fleas counted and removed approximately 48 hours after infestation. Animals were infested on days 12 and 26, and combed and fleas counted and removed approximately 48 hours after infestation. Infestation/counting were repeated approximately monthly until efficacy fell below 80% [% efficacy = (1 - (mean flea count of treatment group ö mean flea count of control group) x 100).

Blood samples were collected from animals in treatment groups on Day 0 at 1, 2, 3 and 6 hours after treatment, on Day 1 at 24 hours after treatment, and whenever emesis was observed. Blood samples were also collected when flea counts were determined. Animals were observed hourly for 6 hours post treatment for emesis.

The active compound provided >90% efficacy against fleas for between 55 and 90 days in animals in group 1. For animals in group 2 >90% efficacy was maintained for up to between 5 and 6 months. For animals in group 3 close to 100% efficacy has been demonstrated for >12 months.

Vomition occurred in group 2 where peak plasma levels were >100ng/ml. No vomition was observed in group 3. Plasma drug levels of dogs in group 3 are shown in Figure 1.

## Claims

1. A use of a compound of formula I: 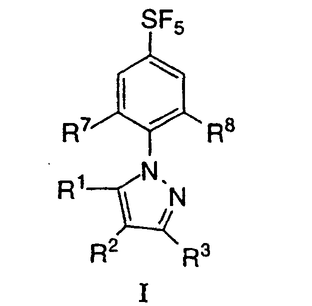 wherein
R¹ is hydrogen, halogen, or a group NR⁴R⁵;
R² is -S(O)ₙR⁶;
R³ is -CN or CX-NY¹Y²;
R⁴ and R⁵ are independently hydrogen or C₁₋₆ straight or branched chain alkyl;
R⁶ is halo C₁₋₆ straight or branched chain alkyl;
R⁷ and R⁸ are independently halogen;
X is O or S;
Y¹ and Y² are independently hydrogen, nitro, amino, or C₁₋₆ straight or branched chain alkyl optionally substituted by halogen, C₃₋₁₀ cycloalkyl, formyl, C₂₋₇ alkanoyl, C₄₋₇ cycloalkylcarbonyl, C₂₋₇ alkoxycarbonyl, or C₂₋₇ haloalkoxycarbonyl; or
Y¹ and Y² together with the nitrogen to which they are attached form an aliphatic heterocyclic group containing from 4 to 8 ring atoms and optionally substituted by halogen or C₁₋₆ straight or branched chain alkyl; or
Y¹ and Y² together form the group =CHY³; or
Y¹ is hydrogen and Y² is C₂₋₇ alkoxycarbonyl, C₁₋₆ straight or branched chain alkylcarbonyl, or -S(O)nR⁶;
Y³ is C₁₋₆ straight or branched chain alkyl, C₂₋₆ alkenyl, or amino optionally substituted by halogen;
n is 0, 1 or 2; or
a pharmaceutically acceptable salt thereof for the manufacture of a medicament for systemic administration for controlling ectoparasitic infestation in a companion animal while avoiding or reducing the potential of vomition.

2. A use of Claim 1 wherein said compound has formula Ia: 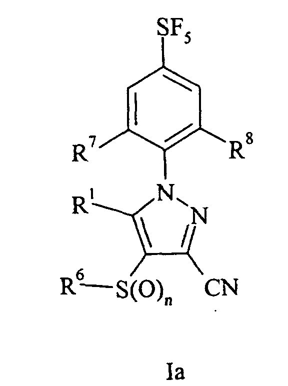 wherein
R¹ is halogen or NR4R5;
R⁴ and R⁵ are independently hydrogen or C₁₋₄ alkyl;
R⁶ is C₁₋₄ haloalkyl;
R⁷ and R⁸ are independently chloro or bromo; and
n is 0, 1 or 2.

3. A use of Claim 2 wherein
R¹ is bromine or NR⁴R⁵;
R⁴ and R⁵ are independently hydrogen or methyl; and
R⁶ is halomethyl or haloethyl.

4. A use of Claim 1 wherein said compound of formula I is selected from the group consisting of:
(1) 6-amino-3-cyano-1-(2,6-dichloro-4-sulfurpentafluorophenyl)-4-(trifluoromethylthio)pyrazole;
(2) 6-amino-3-cyano-1-(2,6-dichloro-4-sulfurpentafluorophenyl)-4-(trifluoromethylsulfinyl)pyrazole;
(3) 6-amino-3-cyano-1-(2,6-dichloro-4-sulfurpentafluorophenyl)-4-(trifluoromethylsulfonyl)pyrazole;
(4) 6-amino-3-cyano-1-(2,6-dichloro-4-sulrurpentafluorophenyl)-4-(1,1-difluoroethylthio)pyrazole; and
(5) 6-bromo-3-cyano-1-(2,6-dichloro-4-sulfurpentafluorophenyl)-4-(trifluoromethylthio)pyrazole.

5. A use of any of Claims 1 to 4 wherein said ectoparasite is flea.

6. A use of any of Claims 1 to 4 wherein said compound of formula I is adminstered parenterally.

7. A use of any preceding claim in which the medicament provides peak plasma level of the administered compound of lower than about 100 ng/ml.

8. A pharmaceutical composition for injection which comprises:
(1) a compound of formula I as defind in any one of claims 1 to 4;
(2) a biologically acceptable polymer; and
(3) a lipophilic solvent.

9. A composition of Claim 8 which comprises:
(1) 1% to 30% of a compound of formula I;
(2) 1% to 20%% w/v of a biodegradable poly (lactide/glycolide) copolymer;
(3) 0 to 90 v/v of a hydrophilic solvent; and
(4) 100 to 10 v/v of a lipophilic solvent.

10. A composition of Claim 8 which comprises:
(1) 1% to 10% of a compound of formula I;
(2) 1% to 10%% w/v of a biodegradable poly (lactide/glycolide) copolymer; and
(3) a mixture of hydrophilic and lipophilic solvents wherein the volume ratio of the hydrophilic and lipophilic solvents is from about 80:20 to about 10:90.

11. A composition of Claim 8 comprising
(1) 5 to 10% w/v of 6-amino-3-cyano-1-(2,6-dichloro-4-sulfurpentafluorophenyl)-4-(trifluoromethylthio)pyrazole;
(2) 5 to 10 % w/v of a biodegradable poly (lactide/glycolide) copolymer; and
(3) glycerol formal:triacetin ratio from 50:50 to 0:100.

## Patentansprüche

1. Eine Verwendung einer Verbindung der Formel I: 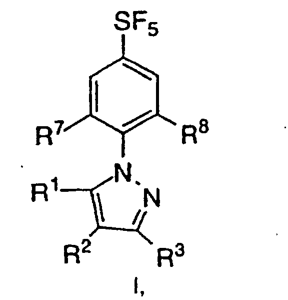 wobei
R¹ Wasserstoff, Halogen oder eine Gruppe NR⁴R⁵ ist,
R² -S(O)ₙR⁶ ist,
R³ -CN oder CX-NY¹Y² ist,
R⁴ und R⁵ unabhängig Wasserstoff oder gerad- oder verzweigtkettiges C₁₋₆-Alkyl sind,
R⁶ gerad- oder verzweigtkettiges Halogen-C₁₋₆-alkyl ist,
R⁷ und R⁸ unabhängig Halogen sind,
X O oder S ist,
Y¹ und Y² unabhängig sind Wasserstoff, Nitro, Amino oder gerad- oder verzweigtkettiges C₁₋₆-Alkyl, gegebenenfalls substituiert durch Halogen, C₃₋₁₀-Cycloalkyl, Formyl, C₂₋₇-Alkanoyl, C₄₋₇-Cycloalkylcarbonyl, C₂₋₇-Alkoxycarbonyl oder C₂₋₇-Halogenalkoxycarbonyl, oder
Y¹ und Y² zusammen mit dem Stickstoff, an den sie gebunden sind, eine aliphatische heterocyclische Gruppe bilden, die 4 bis 8 Ringatome enthält und gegebenenfalls substituiert ist durch Halogen oder gerad- oder verzweigtkettiges C₁₋₆-Alkyl, oder
Y¹ und Y² zusammen die Gruppe =CHY³ bilden oder
Y¹ Wasserstoff ist und Y² C₂₋₇-Alkoxycarbonyl, gerad- oder verzweigtkettiges C₁₋₆-Alkylcarbonyl oder -S(O)ₙR⁶ ist,
Y³ gerad- oder verzweigtkettiges C₁₋₆-Alkyl, C₂₋₆-Alkenyl oder Amino, gegebenenfalls substituiert durch Halogen, ist,
n 0, 1 oder 2 ist, oder
eines pharmazeutisch annehmbaren Salzes davon zur Herstellung eines Medikaments für die systemische Verabreichung zur Bekämpfung eines Ektoparasitenbefalls bei einem Haustier, wobei die Erbrechenswahrscheinlichkeit vermieden oder verringert wird.

2. Eine Verwendung nach Anspruch 1, wobei die Verbindung die Formel la besitzt: 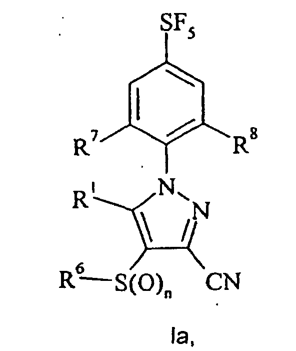 wobei
R¹ Halogen oder NR⁴R⁵ ist,
R⁴ und R⁵ unabhängig Wasserstoff oder C₁₋₄-Alkyl sind,
R⁶ C₁₋₄-Halogenalkyl ist,
R⁷ und R⁸ unabhängig Chlor oder Brom sind und
n 0, 1 oder 2 ist.

3. Eine Verwendung nach Anspruch 2, wobei
R¹ Brom oder NR⁴R⁵ ist,
R⁴ und R⁵ unabhängig Wasserstoff oder Methyl sind und
R⁶ Halogenmethyl oder Halogenethyl ist.

4. Eine Verwendung nach Anspruch 1, wobei die Verbindung der Formel I ausgewählt ist aus der Gruppe, bestehend aus:
(1 ) 6-Amino-3-cyano-1-(2,6-dichlor-4-schwefelpentafluorphenyl)-4-(trifluormethylthio)-pyrazol,
(2) 6-Amino-3-cyano-1-(2,6-dichlor-4-schwefelpentafluorphenyl)-4-(trifluormethylsulfinyl)pyrazol,
(3) 6-Amino-3-cyano-1-(2,6-dichlor-4-schwefelpentafluorphenyl)-4-(trifluormethylsulfonyl)pyrazol,
(4) 6-Amino-3-cyano-1-(2,6-dichlor-4-schwefelpentafluorphenyl)-4-(1,1-difluorethylthio)-pyrazol und
(5) 6-Brom-3-cyano-1-(2,6-dichlor-4-schwefelpentafluorphenyl)-4-(trifluormethylthio)-pyrazol.

5. Eine Verwendung nach irgendeinem der Ansprüche 1 bis 4, wobei der Ektoparasit ein Floh ist.

6. Eine Verwendung nach irgendeinem der Ansprüche 1 bis 4, wobei die Verbindung der Formel I parenteral verabreicht wird.

7. Eine Verwendung nach irgendeinem vorhergehenden Anspruch, wobei das Medikament Plasmaspitzenwerte der verabreichten Verbindung von weniger als etwa 100 ng/ml ergibt.

8. Eine pharmazeutische Zusammensetzung zur Injektion, die enthält:
(1) eine wie in irgendeinem der Ansprüche 1 bis 4 definierte Verbindung der Formel I,
(2) ein biologisch annehmbares Polymer und
(3) ein lipophiles Lösungsmittel.

9. Eine Zusammensetzung nach Anspruch 8, die enthält:
(1) 1% bis 30% einer Verbindung der Formel I,
(2) 1% bis 20% Gew.Nol. eines biologisch abbaubaren Poly(lactid/glycolid)-copolymers,
(3) 0 bis 90 Vol./Vol. eines hydrophilen Lösungsmittels und
(4) 100 bis 10 Vol./Vol. eines lipophilen Lösungsmittels.

10. Eine Zusammensetzung nach Anspruch 8, die enthält:
(1) 1% bis 10% einer Verbindung der Formel I,
(2) 1% bis 10% Gew.Nol. eines biologisch abbaubaren Poly(lactid/glycolid)-copolymers und
(3) eine Mischung aus hydrophilen und lipophilen Lösungsmitteln, wobei das Volumenverhältnis der hydrophilen und der lipophilen Lösungsmittel etwa 80:20 bis etwa 10:90 beträgt.

11. Eine Zusammensetzung nach Anspruch 8, umfassend
(1) 5 bis 10% Gew.Nol. 6-Amino-3-cyano-1-(2,6-dichlor-4-schwefelpentafluorphenyl)-4-(trifluormethylthio)pyrazol,
(2) 5 bis 10% Gew./Vol. eines biologisch abbaubaren Poly(lactid/glycolid)-copolymers und
(3) Glycerinformal:Triacetin im Verhältnis 50:50 bis 0:100.

## Revendications

1. Utilisation d'un composé de formule I : 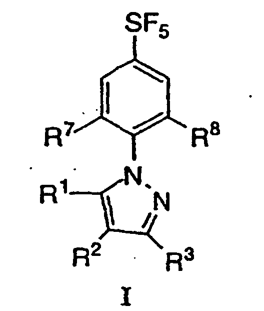 dans laquelle
R¹ est un atome d'hydrogène ou d'halogène, ou un groupe NR⁴R⁵ ;
R² est -S(O)ₙR⁶ ;
R³ est -CN ou CX-NY¹Y² ;
R⁴ et R⁵ sont indépendamment un atome d'hydrogène ou un groupe alkyle en C₁₋₆ à chaîne linéaire ou ramifiée ;
R⁶ est un groupe halogénoalkyle en C₁₋₆ à chaîne linéaire ou ramifiée ;
R⁷ et R⁸ sont indépendamment des atomes d'halogène ;
X est O ou S ;
Y¹ et Y² sont indépendamment des atomes d'hydrogène ou des groupes nitro, amino, ou alkyle en C₁₋₆ à chaîne linéaire ou ramifiée éventuellement substitué par un atome d'halogène ou un groupe cycloalkyle en C₃₋₁₀, formyle, alcanoyle en C₂₋₇, cycloalkylcarbonyle en C₄₋₇, alcoxycarbonyle en C₂₋₇ ou halogénoalcoxycarbonyle en C₂₋₇, ou
Y¹ et Y², conjointement avec l'atome d'azote auquel ils sont fixés, forment un groupe hétérocyclique aliphatique contenant de 4 à 8 atomes dans le noyau et éventuellement substitué par un atome d'halogène ou un groupe alkyle en C₁₋₆ à chaîne linéaire ou ramifiée ; ou
Y¹ et Y² forment ensemble le groupe =CHY³ ; ou
Y¹ est un atome d'hydrogène et Y² est un groupe alcoxycarbonyle en C₂₋₇, alkyl(en C₁₋₆ à chaîne linéaire ou ramifiée)carbonyle ou -S(O)ₙR⁶ ;
Y³ est un groupe alkyle en C₁₋₆ à chaîne linéaire ou ramifiée ; alcényle en C₂₋₆ ou amino éventuellement substitué par un atome d'halogène ;
n vaut 0, 1 ou 2 ; ou
sel pharmaceutiquement acceptable de ce composé, pour la fabrication d'un médicament à administrer par voie générale, destiné à lutter contre une infestation d'ectoparasites chez un animal de compagnie tout en évitant ou en réduisant le potentiel de vomissements.

2. Utilisation selon la revendication 1 dans laquelle ledit composé a pour formule Ia : 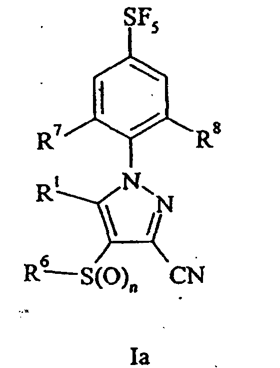 dans laquelle
R¹ est un atome d'halogène ou un groupe NR⁴R⁵ ;
R⁴ et R⁵ sont indépendamment des atomes d'hydrogène ou des groupes alkyle en C₁₋₄ ;
R⁶ est un groupe halogénoalkyle en C₁₋₄ ;
R⁷ et R⁸ sont indépendamment des groupes chloro ou bromo ; et
n vaut 0, 1 ou 2.

3. Utilisation selon la revendication 2 dans laquelle
R¹ est un atome de brome ou un groupe NR⁴R⁵ ;
R⁴ et R⁵ sont indépendamment des atomes d'hydrogène ou des groupes méthyle ; et
R⁶ est un groupe halogénométhyle ou halogénoéthyle.

4. Utilisation selon la revendication 1 dans laquelle ledit composé de formule I est choisi dans le groupe constitué par :
(1) le 6-amino-3-cyano-1-(2,6-dichloro-4-sulfopentafluorophényl)-4-(trifluorométhylthio)pyrazole ;
(2) le 6-amino-3-cyano-1-(2,6-dichloro-4-sulfopentafluorophényl)-4-(trifluorométhylsulfinyl)pyrazole ;
(3) le 6-amino-3-cyano-1-(2,6-dichloro-4-sulfopentafluorophényl)-4-(trifluorométhylsulfonyl)pyrazole ;
(4) le 6-amino-3-cyano-1-(2,6-dichloro-4-sulfopentafluorophényl)-4-(1,1-difluoroéthylthio)pyrazole ; et
(5) le 6-bromo-3-cyano-1-(2,6-dichloro-4-sulfopentafluorophényl)-4-(trifluorométhylthio)pyrazole.

5. Utilisation selon l'une quelconque des revendications 1 à 4 dans laquelle ledit ectoparasite est la puce.

6. Utilisation selon l'une quelconque des revendications 1 à 4 dans laquelle ledit composé de formule I est administré par voie parentérale.

7. Utilisation selon l'une quelconque des revendications précédentes dans laquelle le médicament permet d'atteindre un pic plasmatique du composé administré de moins d'environ 100 ng/ml.

8. Composition pharmaceutique pour injection qui comprend :
(1) un composé de formule I tel que défini dans l'une quelconque des revendications 1 à 4 ;
(2) un polymère acceptable sur le plan biologique ; et
(3) un solvant lipophile.

9. Composition selon la revendication 8 qui comprend :
(1) 1 % à 30% d'un composé de formule I ;
(2) 1% à 20% en poids/volume d'un copolymère poly-(lactide/glycolide) biodégradable ;
(3) 0 à 90 en volume d'un solvant hydrophile ; et
(4) 100 à 10 en volume d'un solvant lipophile.

10. Composition selon la revendication 8 qui comprend :
(1) 1 % à 10% d'un composé de formule I ;
(2) 1% à 10% en poids/volume d'un copolymère poly-(lactide/glycolide) biodégradable ; et
(3) un mélange de solvants hydrophiles et lipophiles dans lequel le rapport volumique des solvants hydrophiles aux solvants lipophiles est d'environ 80 : 20 à environ 10 : 90.

11. Composition selon la revendication 8 comprenant
(1) 5 à 10% en poids/volume de 6-amino-3-cyano-1-(2,6-dichloro-4-sulfopentafluorophényl)-4-(trifluorométhylthio)pyrazole ;
(2) 5 à 10 % en poids/volume d'un copolymère poly-(lactide/glycolide) biodégradable ; et
(3) un rapport glycérolformal : triacétine de 50 : 50 à 0 : 100.
